# EUROPEAN PATENT SPECIFICATION

(11) **EP 2 815 777 B1**
(45) Date of publication and mention of the grant of the patent: **18.01.2017**
(21) Application number: 13749702.0
(22) Date of filing: 14.02.2013
(51) Int. Cl.: A61M 3/00, A61C 17/02, A61C 17/00, A61B 1/12, A61M 3/02

(54) **WATER JET SPRAY NOZZLE, WATER JET SPRAY DEVICE, GASTROINTESTINAL TRACT CLEANING DEVICE, AND GASTRIC WALL CLEANING DEVICE**
WASSERSTRAHLSPRÜHDÜSE, WASSERSTRAHLSPRÜHVORRICHTUNG, REINIGUNGSVORRICHTUNG FÜR MAGEN-DARM-TRAKT UND MAGENWANDREINIGUNGSVORRICHTUNG
BUSE DE PULVÉRISATION À JET D'EAU, DISPOSITIF DE PULVÉRISATION À JET D'EAU, DISPOSITIF DE NETTOYAGE DE TRACTUS GASTRO-INTESTINAL, ET DISPOSITIF DE NETTOYAGE DE PAROI GASTRIQUE

(30) Priority: 14.02.2012 JP 2012029019
(43) Date of publication of application: 24.12.2014
(73) Proprietor: Tohoku University, Sendai-shi, Miyagi 980-8577 (JP); Showa University, Tokyo 142-8555 (JP)
(72) Inventor: SOYAMA, Hitoshi, Sendai-shi Miyagi 980-8577 (JP); AMI, Katsunori, Tokyo 173-0015 (JP); YAMAMOTO, Matsuo, Tokyo 142-8555 (JP)
(74) Representative: Plougmann Vingtoft a/s
(86) International application number: PCT/JP2013/053480
(87) International publication number: WO 2013/122125

(56) References cited:
- WO-A1-2004/052338
- JP-A- H10 192 307
- US-A- 6 139 319
- US-A1- 2011 028 887
- Russell Nielsen ET AL: "Effect of cavitation on spray formation", , 1 February 1960 (1960-02-01), XP055172671, Retrieved from the Internet: URL:http://deepblue.lib.umich.edu/bitstrea m/handle/2027.42/6824/bac9790.0001.001.pdf ?sequence=5 [retrieved on 2015-02-27]
- Satoshi Nishimura ET AL: "Similarity Law on Shedding Frequency of Cavitation Cloud Induced by a Cavitating Jet", journal of fluid science and technology, vol. 7, no. 3, 1 January 2012 (2012-01-01) , pages 405-420, XP055297231, JP ISSN: 1880-5558, DOI: 10.1299/jfst.7.405

## Description

### Technical Field

The present invention relates to a water jet spray nozzle, a water jet spray device, a cleaning method using a water jet, a gastrointestinal tract cleaning device, and a gastric wall cleaning device.

### Background Art

In recent years, the creation of cleaning methods using water jets with low discharge pressures has been demanded in the fields of medicine and engineering. For example, in order to remove gastric mucus interrupting a detailed endoscopic observation of an upper part, a gastric wall is sprayed and cleaned by a water jet on an endoscope (For example, see Non Patent Literature 1).

Russell Nielsen et al. ("Effect of cavitation on spray formation", Progress report, UMRI Project; February 1960, The University of Michigan Research Institute, Ann Arbor) discloses a cavitating venture nozzle. Experiments conducted with the nozzle indicate that cavitating performance may be obtained and duplicated. Whether at threshold of cavitation or beyond it, the implosion of vapor bubbles nucleated at the throat of venture is shown to affect and perturb the jet issuing from the nozzle where the process of spray formation originates. US6139319 (A) relates to a jet nozzle for a hand piece of an oral irrigator, which has a nozzle head in which a feed line runs. A feed line opens into an outlet orifice from which a liquid can emerge in the form of a single jet. A constriction is provided in the feed line. The constriction acts to produce a large number of micro fine gas bubbles. The micro fine gas bubbles are better suited to penetrate deep into subcrestal pockets in order to kill anaerobic bacteria which have settled there by the supply of oxygen, hence treating and curing inflammations of the gums.

US2011028887 (A1) relates to a water jet surgical instrument including a supply line for supplying a cutting fluid and a discharge nozzle for forming and discharging a fluid jet. The fluid jet has a predetermined spread angle and/or a predetermined emergence energy. A jet shaping device is arranged relative to the discharge nozzle such that the fluid jet is able to be adjusted by the jet shaping device with regard to the spread angle and/or emergence energy thereof.

The present inventors have found that the use of gradually contracting/expanding tubes such as a venturi tube can sufficiently developed cavitation even with a low discharge pressure (For example, see Non Patent Literatures 2 to 5).

### Citation List

### Non Patent Literatures

[Non Patent Literature 1]: "Full Digital Electronic Endoscope System Advancia", FUJIFILM Medical Co., Ltd., p. 10
[Non Patent Literature 2]: Soyama and Muraoka, "Generation of Hydroxyl Radical by Fluid Cavitation", The 45th JSME Autumn Lecture Collection, 2009, p.49-50
[Non Patent Literature 3]: Soyama, H. and Muraoka, T., "Chemical Reactor Using Radical Induced by a Cavitating Jet", Proceedings of 20th International Conference on Water Jetting, 2010, p.259-267
[Non Patent Literature 4]: Muraoka and Soyama, "Zooplankton Killing by Fluid Cavitation", JSME Hydraulic Engineering Department Lecture Collection, 2010, p.153-154
[Non Patent Literature 5]: Soyama and Muraoka, "Pressure Influence of Cavitation Collapse Field in Light Emission by Fluid Cavitation", Cavitation Symposium (15th), 2010, A2-1, p.1-4

### Summary of the Invention

### Technical Problem

In cleaning with the water jet described in Non Patent Literature 1, a pipe line for feeding a liquid to the end of the endoscope has a small diameter with a large pressure loss. Thus, sufficient cleaning capability cannot be obtained. If the water jet has a high water pressure, an impact force applied from the water jet concentrates in a narrow range. Thus, the water jet used for cleaning a gastric wall may cause damage on gastric tissues, e.g., gastric mucosa. Thus, a water pressure needs to be reduced during cleaning and thus may leave gastric mucus with insufficient cleaning capability.

The present invention has been devised in view of this problem. An object of the present invention is to provide a water jet spray nozzle, a water jet spray device, a cleaning method using the water jet, a gastrointestinal tract cleaning device, and a gastric wall cleaning device, which can improve cleaning capability relative to a water pressure and effectively remove gastric mucus without damaging gastric tissues, e.g., gastric mucosa when the present invention is used for cleaning a gastric wall.

### Solution to Problem

A water jet spray nozzle according to the present invention is defined by claim 1 and has a flow path including, from the upstream side
to the end of the flow path, a small-diameter portion having a smaller inside diameter than the flow path upstream of the small-diameter portion , an expanded portion having a larger inside diameter than the small-diameter portion, and a jet orifice having a smaller inside diameter than the expanded portion, the water jet spray nozzle being configured such that water passing through the flow path with a predetermined pressure is retained in the expanded portion so as to generate cavitation bubbles from the small-diameter portion to the expanded portion.
The inside diameter of the jet orifice is 1 to 1.3 times larger than the inside diameter of the small-diameter portion and the inside diameter of the small-diameter portion is 0.2 to 1.0 mm.

According to the water jet spray nozzle of the present invention, water passing through the flow path with the predetermined pressure is retained in the expanded portion so as to generate cavitation bubbles from the small-diameter portion to the expanded portion. Moreover, a water jet can be discharged from the jet orifice on the end of the flow path. At this point, generated cavitation causes pressure fluctuations on the water jet so as to interrupt a jet core. This can increase an impact force caused by collision of water jet droplets and liquids and expand the range of a water jet coming into contact with the surface of a cleaned object. Thus, a larger impact force can be generated over a wider range than that of a typical water jet, improving the cleaning capability relative to a water pressure.

In this case, a jet is a discharged state of a mixed phase of a gas phase part including cavitation and air and a liquid phase part including liquid columns, liquids, and droplets, a cavitating jet includes a mixed phase of cavitation and a liquid phase, a water jet includes a mixed phase of air and a liquid phase, and the jet part of a liquid column is simply called a water jet.

The water jet spray nozzle according to the present invention can be properly used if effective cleaning is necessary while preventing damage on the surface of the cleaned object, for example, in the cleaning of a gastric wall that is a gastrointestinal tract or removal of a biofilm in a mouth. The water jet spray nozzle according to the present invention has high cleaning capability even at a relatively low water pressure and can widely disperse the impact force of a water jet. Thus, the water jet spray nozzle used for cleaning a gastric wall or a mouth can effectively remove gastric mucus, a biofilm, and so on without damaging gastric or oral tissues.

A cleaning method using a conventional cavitating jet can obtain high cleaning effect with a low discharge pressure but requires the surface of a cleaned object to be immersed with water. In contrast, the water jet spray nozzle according to the present invention can obtain the same cleaning effect as a cavitating jet without immersing the surface of the cleaned object with water. The water jet spray nozzle according to the present invention can be used for cleaning locations that are hard to immerse with water, promising a wider range of uses than that of a cavitating jet.

According to the water jet spray nozzle of the present invention, the inside diameter of the jet orifice is preferably 1 to 1.3 times larger than the inside diameter of the small-diameter portion. In this case, water passing through the flow path with the predetermined pressure can be effectively retained in the expanded portion. According to the water jet spray nozzle of the present invention, the inside diameter of the small-diameter portion is preferably 0.2 to 1.0 mm, particularly, 0.5 to 0.7 mm. In this case, when water passes through the flow path with the predetermined pressure, cavitation bubbles can be effectively generated from the small-diameter portion to the expanded portion. Thus, in these cases, the cleaning capability can be further improved. Furthermore, in order to retain water in the expanded portion and effectively generate cavitation bubbles, water passing through the flow path preferably has a pressure of 0.2 to 0.6 MPa.

A water jet spray device according to the present invention includes a water jet spray nozzle according to the present invention, and a supply unit that supplies water to the water jet spray nozzle with the predetermined pressure.

The water jet spray device according to the present invention includes the water jet spray nozzle according to the present invention. Thus, a larger impact force can be generated over a wider range than that of a typical water jet, improving the cleaning capability relative to a water pressure. The water jet spray device can be properly used for effective cleaning while preventing damage on the surface of a cleaned object in, for example, cleaning of a gastric wall, removal of a biofilm in a mouth, cleaning of cuts (cleaning of wounds), removal of dirt under nails (finger tip cleaning), and removal of sebum from hair roots.

According to the water jet spray device of the present invention, the supply unit is preferably configured to supply water with a pressure of 0.2 to 0.6 MPa. In this case, the cleaning effect of a water jet can be particularly improved.

A cleaning method using a water jet according to the present invention, including discharging the water jet from the jet orifice to the surface of a cleaned object by means of the water jet spray device according to the present invention with a distance of 1.5 to 11 mm between the jet orifice and the surface of the cleaned object.

According to the cleaning method using a water jet of the present invention, a distance between the jet orifice and the surface of the cleaned object is 1.5 to 11 mm. This can perform particularly effective cleaning using the water jet spray device according to the present invention.

A gastrointestinal tract cleaning device includes: the water jet spray nozzle according to the present invention; and a supply unit that supplies water to the water jet spray nozzle with a pressure of 0.2 to 0.6 MPa.

The gastrointestinal tract cleaning device and the gastric wall cleaning device according to the present invention includes the water jet spray nozzle according to the present invention. Thus, a larger impact force can be generated over a wider range than that of a typical water jet, improving the cleaning capability relative to a water pressure. This cleans off a gastrointestinal tract such as a gastric wall at a relatively low water pressure, thereby effectively removing gastric mucus, and so on without damaging gastric tissues, e.g., gastric mucosa. According to the gastrointestinal tract cleaning device and the gastric wall cleaning device of the present invention, the inside diameter of the small-diameter portion of the water jet spray nozzle is 0.2 to 1.0 mm. In this case, a gastrointestinal tract such as a gastric wall can be particularly effectively cleaned.

According to the water jet spray nozzle, the water jet spray device, the cleaning method using a water jet, a gastrointestinal tract cleaning device, and a gastric wall cleaning device of the present invention, gastric mucus, a biofilm, and so on can be removed effectively so as to be collected from a cleaning solution after the cleaning. Thus, the cleaning solution is collected after the cleaning and is centrifugally separated to observe bacteria and cells in a precipitate. This can precisely identify Helicobacter pylori contained in gastric mucus, a biofilm, and so on and diagnose the presence of a cancer. Furthermore, bacteria can be effectively cultivated using the collected bacteria, thereby increasing the accuracy of identification of bacteria. Conventionally, it is difficult to collect a sufficient amount of gastric mucus, a biofilm, and so on from a deep layer, leading to difficulty in identification of Helicobacter pylori and diagnosis of cancers.

### Advantageous Effect of Invention

The present invention can provide a water jet spray nozzle, a water jet spray device, a cleaning method using a water jet, a gastrointestinal tract cleaning device, and a gastric wall cleaning device, which can improve cleaning capability relative to a water pressure and effectively remove gastric mucus without damaging gastric tissues, e.g., gastric mucosa when the present invention is used for cleaning a gastric wall.

### Brief Description of the Drawings

[FIG. 1] FIG. 1 is a side view of a water jet spray device according to an embodiment of the present invention.
[FIG. 2] FIG. 2 shows side views of (a) nozzle B and (b) nozzle C that are used as comparative examples in ejection experiments of the water jet spray device according to the embodiment of the present invention.
[FIG. 3] FIG. 3(a) is a table of impact patterns formed by jets onto impact paper with varying standoff distances in the ejection experiments of the water jet spray device according to the embodiment of the present invention, and
FIG. 3(b) shows micrographs of histopathological images of a gastric wall in cross section in HE staining (upper) and PAS staining (lower) before and after cleaning of the gastric wall.
[FIG. 4] FIG. 4 shows moment photographs of (a) a jet of a nozzle A and (b) a jet of the nozzle B when a discharge pressure from a pump is changed in ejection experiments of the water jet spray device according to the embodiment of the present invention.
[FIG. 5] FIG. 5 shows moment photographs of (a) a jet of the nozzle A and (b) a jet of the nozzle B when a discharge pressure p₁ from the pump is 0.5 MPa in ejection experiments of the water jet spray device in FIG. 4.
[FIG. 6] FIG. 6 shows photographic strips on an ejected state of a jet of the nozzle A when the discharge pressure p₁ from the pump is 0.3 MPa in ejection experiments of the water jet spray device in FIG. 4.
[FIG. 7] FIG. 7 shows (a) a micrograph of a histopathological image at an uncleaned point on a gastric wall of a miniature pig and (b) a micrograph of a histopathological image at a cleaned point on the gastric wall of the miniature pig during cleaning of gastric mucus of the miniature pig by means of the water jet spray nozzle according to the embodiment of the present invention.

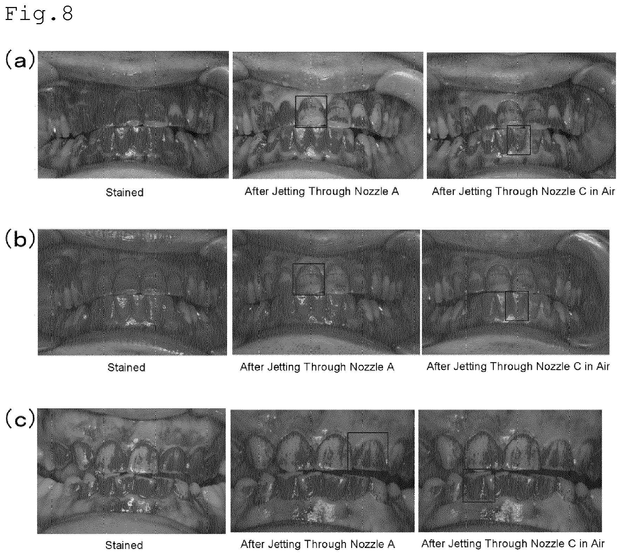

[FIG. 8] FIG. 8 (a) is an observation photograph of a surface condition of stained teeth after a water jet discharge to the right incisor of an upper jaw through the nozzle A and a water jet discharge to the right incisor of a lower jaw through the nozzle C, FIG. 8 (b) is an observation photograph of a surface condition of stained teeth after a water jet discharge to the right incisor of the upper jaw through the nozzle A and a water jet discharge to the left incisor of the lower jaw through the nozzle C, and FIG. 8(c) is an observation photograph of a surface condition of stained teeth after a water jet discharge to the left incisor of the upper jaw through the nozzle A and a water jet discharge to the right incisor of the lower jaw through the nozzle C, during cleaning of a biofilm in a mouth by means of the water jet spray nozzle according to the embodiment of the present invention.
[FIG. 9] FIG. 9 (a) is an observation photograph of a surface condition of a stained titanium test piece after a water jet discharge through the nozzle A, and FIG. 9(b) is an observation photograph of a surface condition of a stained titanium test piece from a different subject after a water jet discharge through the nozzle A, during cleaning of the titanium test piece collected after being held in a mouth by means of the water jet spray nozzle according to the embodiment of the present invention.

### Description of Embodiments

An embodiment of the present invention will be described below with reference to the accompanying drawings.

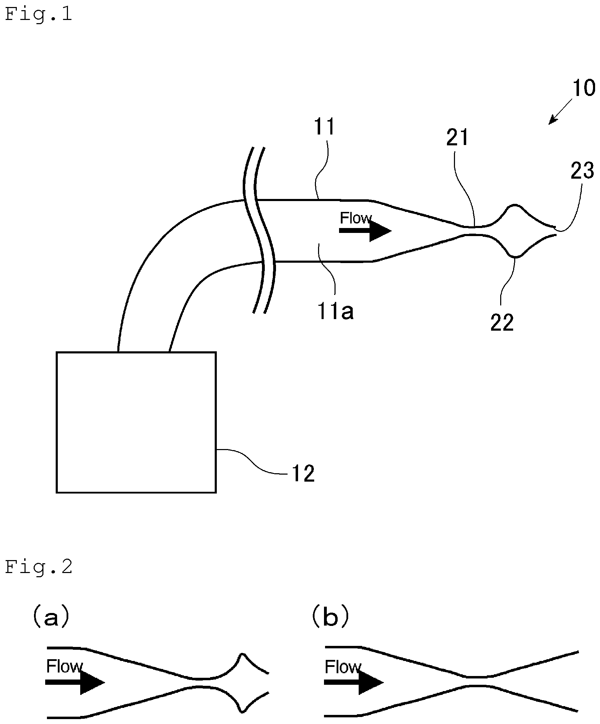

FIGS. 1 to 6 show a water jet spray nozzle, a water jet spray device, a cleaning method using the water jet, a gastrointestinal tract cleaning device, and a gastric wall cleaning device according to the embodiment of the present invention.

A water jet spray device 10 according to the embodiment of the present invention includes a water jet spray nozzle 11 and a supply unit 12.

As shown in FIG. 1, the water jet spray nozzle 11 has a flow path 11a including, from the upstream side to the end, a small-diameter portion 21 having a small inside diameter, an expanded portion 22 having a larger inside diameter than the small-diameter portion 21, and a jet orifice 23 having a smaller inside diameter than the expanded portion 22. The flow path 11a gradually tapers down toward the small-diameter portion 21 from the upstream side, gradually expands from the small-diameter portion 21 to the expanded portion 22, and gradually contracts from the expanded portion 22 to the jet orifice 23. The water jet spray nozzle 11 has an inside diameter of 0.2 mm to 1.0 mm on the small-diameter portion 21. The inside diameter of the jet orifice 23 is 1 to 1.3 times larger than that of the small-diameter portion 21.

The supply unit 12 is composed of a pump connected to the upstream side of the water jet spray nozzle 11. The supply unit 12 is configured to supply water to the water jet spray nozzle 11 with a pressure of 0.25 MPa to 0.6 MPa. The water jet spray device 10 retains water in the expanded portion 22 when pressurized water is supplied into the flow path 11a of the water jet spray nozzle 11 from the supply unit 12, thereby generating cavitation bubbles from the small-diameter portion 21 to the expanded portion 22.

The water jet spray device 10 can perform cleaning according to the cleaning method using the water jet according to the embodiment of the present invention. During cleaning, a distance between the jet orifice 23 and the surface of a cleaned object is set at 1.5 to 11 mm. A water jet is sprayed from the jet orifice 23 to the surface of the cleaned object. At this point, cavitation bubbles are generated from the small-diameter portion 21 to the expanded portion 22. The cavitation causes pressure fluctuations on the water jet so as to interrupt a jet core. This can increase an impact force caused by collision of water jet droplets and liquids and expand the range of a water jet coming into contact with the surface of the cleaned object. Thus, a larger impact force can be generated over a wider range than that of a typical water jet, improving the cleaning capability relative to a water pressure.

The water jet spray device 10 can be properly used for effective cleaning while preventing damage on the surface of a cleaned object in, for example, cleaning of a gastric wall, removal of a biofilm in a mouth, cleaning of cuts (cleaning of wounds), removal of dirt under nails (finger tip cleaning), and removal of sebum from hair roots. The water jet spray device 10 has high cleaning capability even at a relatively low water pressure and can widely disperse the impact force of the water jet. Thus, the water jet spray device 10 used for cleaning a gastric wall or a mouth can effectively remove gastric mucus, a biofilm, and so on without damaging gastric or oral tissues. In the cleaning of gastrointestinal tracts such as a gastric wall, the water jet spray device 10 is configured as a gastrointestinal tract cleaning device or a gastric wall cleaning device according to the embodiment of the present invention.

### [Ejection Experiment]

An impact force of the water jet spray nozzle 11, a factor of the cleaning capability was evaluated using impact paper, and a jet flow pattern was observed. Since an impact force during cavitation bubble collapse is regarded as a factor of the cleaning effect of a cavitating jet, it is understood that the cleaning capability can be evaluated by evaluating an impact force.

Sample water pressurized by the pump (a maximum discharge pressure of 0.6 MPa, a maximum discharge flow rate of 7 l/min) of the supply unit 12 was sprayed from the water jet spray nozzle 11 (hereinafter, will be called "nozzle A (Nozzle A) ") shown in FIG. 1. Injection nozzles for comparison under the same conditions were: a nozzle B in FIG. 2 (a) and a nozzle C (Nozzle C) in FIG. 2(b). The inside diameter of the nozzle B is about 1.5 times larger than that of the small-diameter portion. The expanded portion of the nozzle C has a maximum diameter at the jet orifice.

The nozzles are all made of silica glass. The flow paths gradually taper down from the upstream sides to the small-diameter portions and gradually expand from the small-diameter portions to the expanded portions. A diameter d of the small-diameter portion was determined for each of the nozzles by measuring a flow rate. The diameter d of the nozzle A was 0.55 mm, the diameter d of the nozzle B was 0.62 mm, and the diameter d of the nozzle C was 0.62 mm. The patterns of jets generated using the nozzles were captured by moment photography using a Xenon flash lamp with a flash time of 1.1 µs and were observed using a high-speed video camera (a recording speed of 38, 500 frames/second and a shutter speed of 2 µs during the photography).

An impact force generated by each jet was measured by measuring each scanned jet on impact paper (FUJIFILM Corporation, ultra-low pressure LLLW) contained with a stainless board in a polyethylene bag having a thickness of 0.05 mm. Each jet was scanned five times with a scanning speed of 1 . 62 mm/s. The sample water was ion exchanged water. Moreover, a cavitating jet was sprayed into water in an experiment while a water jet was sprayed into air in an experiment.

### [Impact force (cleaning capability) experimental results]

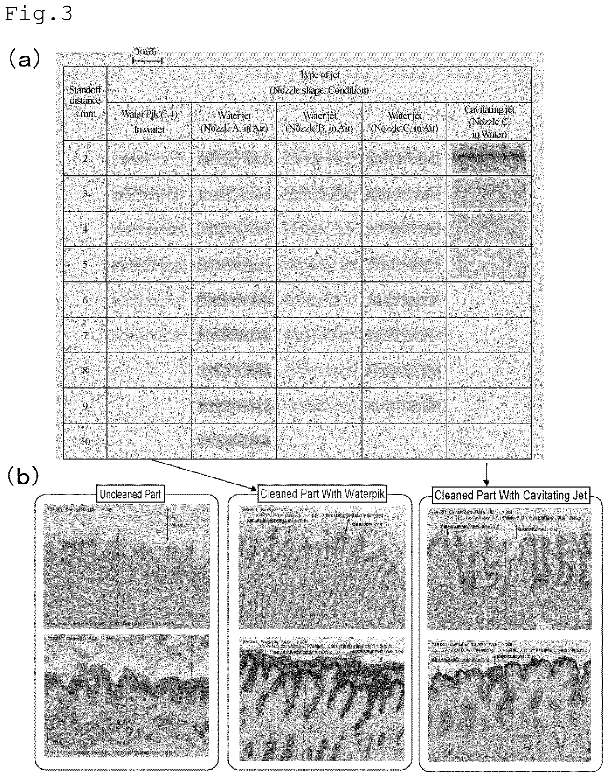

For clarification of the range of a generated impact force of each jet, FIG. 3(a) shows impact patterns in ejections onto impact paper with varying distances (standoff distance) between the impact paper and the nozzles A, B, and C. A discharge pressure p₁ from the pump was 0.3 MPa. For comparison, FIG. 3 (a) also shows discharge results using Water Pik (registered trademark). FIG. 3(b) shows histopathological images of gastric wall conditions when gastric walls are cleaned with Water Pik and the cavitating jet of the nozzle C. Moreover, the standoff distance s is about 3 mm when the gastric walls are cleaned.

As shown in FIG. 3(a), it was confirmed that a jet into the air through the nozzle A increases an impact force with the distance s and expands the range of the impact force. Furthermore, it was confirmed that a jet into the air through Water Pik, the nozzle B, and the nozzle C generates a small impact force in a small range. Moreover, it was confirmed that a cavitating jet into water through the nozzle C can generate a larger impact force over a wider range than in the case of a water jet if the distance s is 2 mm. Thus, like a cavitating jet, a water jet of the nozzle A into the air can generate a larger impact force over a wider range than in the case of a water jet through the nozzles B and C.

As shown in FIG. 3(b), in gastric wall cleaning using Water Pik, the epithelium of gastric mucosa is completely kept to the innermost side but a mucous layer is left in a compacted state. This is because a water jet from Water Pik concentrates in a narrow range. In contrast, in gastric wall cleaning using a cavitating jet through the nozzle C, the epithelium of gastric mucosa is completely kept to the innermost side while a mucous layer completely disappears. Hence, the use of a cavitating jet can obtain an enhanced cleaning effect even with a low discharge pressure. As shown in FIG. 3 (a), a water jet of the nozzle A has a large impact force over a wide range like a cavitating jet of the nozzle C. Thus, the use of a water jet of the nozzle A can also obtain the same cleaning effect as a cavitating jet. Moreover, a water jet of the nozzle A eliminates the need for immersing the surface of a cleaned object with water. Hence, water does not need to be retained in a stomach, reducing the burden of a patient with usability.

### [Jet Observation Results]

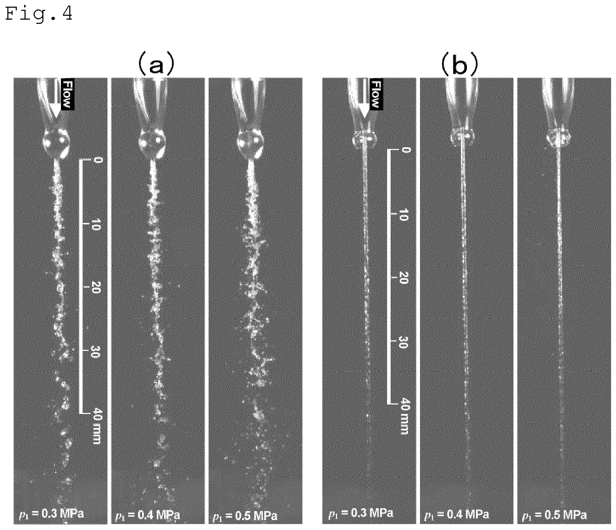

FIG. 4 shows the results of jet patterns captured by moment photography when a jet is discharged into the air through the nozzles A and B with the discharge pressure p₁ changing from 0.3 to 0.5 MPa. As shown in FIG. 4(a), the use of the nozzle A disperses droplets and liquids near a nozzle exit, whereas in the case of the nozzle B, it was confirmed that liquid columns are kept relatively to the downstream side as shown in FIG. 4(b).

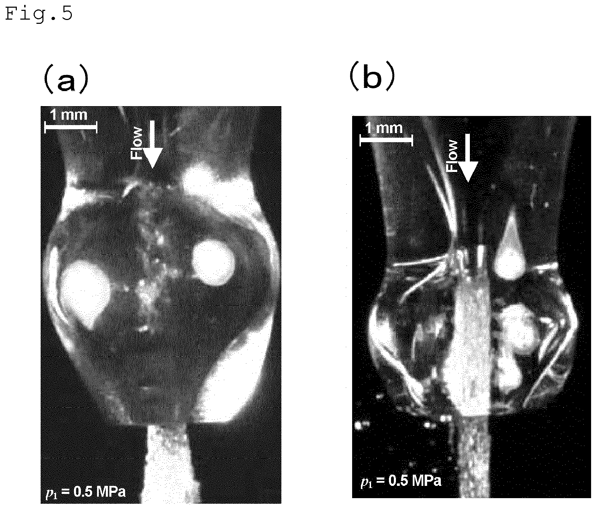

For clarification of a difference between the nozzle A and the nozzle B, FIG. 5 shows photographs of an enlarged nozzle part when the discharge pressure p₁ is 0.5 MPa. As shown in FIG. 5(a), it is confirmed that the expanded portion 22 of the nozzle A is filled with water and generates cavitation. In the case of the nozzle B, as shown in FIG. 5(b), the expanded portion does not retain water and thus a water jet simply passes through the expanded portion.

The results of FIGS. 4 and 5 prove that the expanded portion 22 is filled with water in the nozzle A and thus cavitation intermittently appears. When a jet is discharged from the jet orifice 23, a water jet is likely to slit into droplets or liquids with higher unsteadiness. The split may widely generate an impact force using a jet from the nozzle A. If a space like the expanded portion 22 is provided near the nozzle exit, a natural frequency is generated according to the size of the space. The variable components of the frequency may contribute to the split of the liquid columns of the water jet.

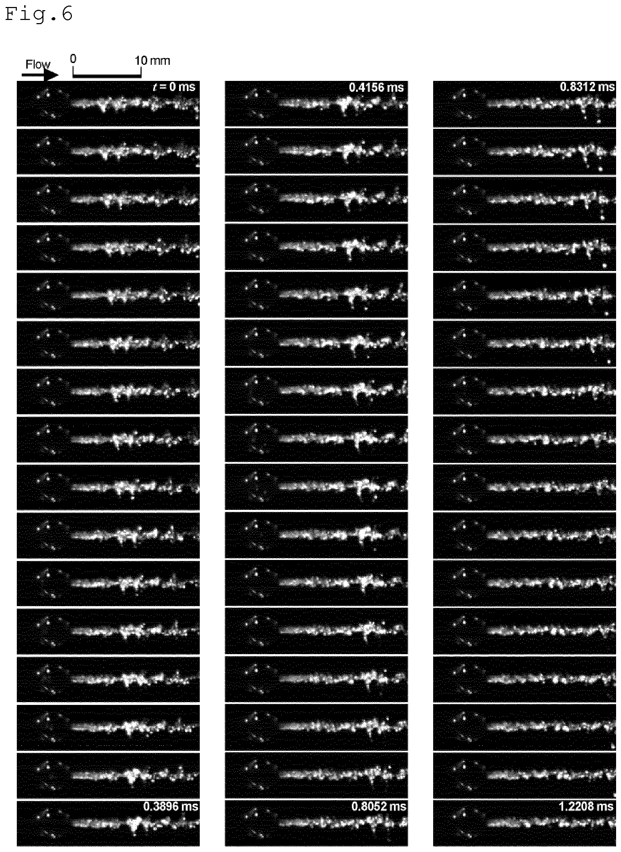

For clarification of the unsteady patterns of a water jet from the nozzle A, FIG. 6 shows results captured by a high-speed video camera. In an experiment, a jet is discharged downward from the nozzle and thus the jet passes from the left to right in FIG. 6. As shown in FIG. 6, liquid columns are relatively kept in a range of several millimeters from the jet orifice 23. It can be confirmed that water jet droplets and liquids are rapidly expand or dispersed toward the downstream side. FIG. 6 proves that a flow velocity at a standoff distance of about 10 mm is about 10 m/s. Since the discharge pressure is 0.3 MPa, a water jet may be expanded and decelerated in the expanded portion 22 provided downstream of the small-diameter portion 21 and discharged from the jet orifice 23. At t of 0 to 0.3896 ms in FIG. 6, wavy patterns at s of 4 to 10 mm indicate that waves are spaced at intervals of about 2 mm with a flow velocity of 10 m/s. Thus, the frequency of the waves is estimated at about 5 kHz. This may generate droplets or liquids from a jet from the nozzle A at several kHz.

### [Example 1]

A tube with the nozzle A in FIG. 1, the water jet spray nozzle 11 according to the embodiment of the present invention, was inserted into the forceps hole of an endoscope such that the nozzle A was attached to the end of the tube, and then a physiological saline was discharged from the nozzle A to clean the gastric mucus of a miniature pig. At this point, the saline was directly discharged in the air to the gastric wall without retained water in the stomach of the miniature pig. The nozzle A has an inside diameter of 1 mm at the small-diameter portion and a nozzle inside diameter of 1.3 mm. The used tube has an outside diameter of 2 mm, an inside diameter of 1.12 mm, and a length of 1.5 m. A discharge pressure upstream of the tube was 0.8 MPa when the physiological saline was discharged. A measurement of a pressure loss of the tube proved that the nozzle A had a discharge pressure of 0.3 MPa downstream of the tube.

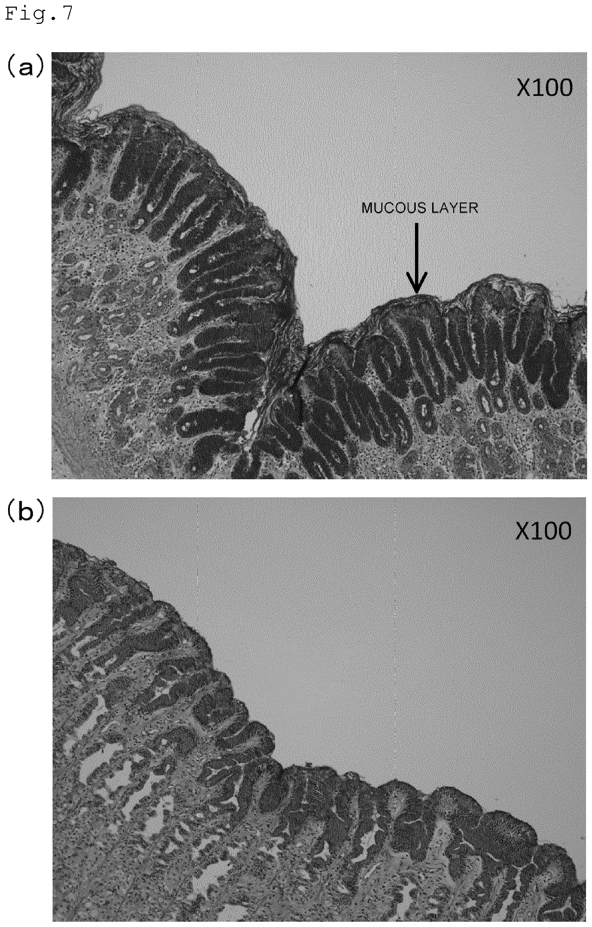

The physiological saline was discharged into the stomach of the miniature pig from the nozzle A to clean the gastric mucus, and then the stomach was collected to capture an image of the condition of the gastric wall. FIG. 7 shows a histopathological image of the gastric wall. FIG. 7 (a) shows a histopathological image of an uncleaned area. FIG. 7(b) is a histopathological image of a cleaned area. As shown in FIG. 7, it was confirmed that the water jet spray nozzle 11 according to the embodiment of the present invention can remove gastric mucus by means of an endoscope and so on. It was further confirmed that the burden of a patient can be reduced because the gastric mucus was cleaned without water retained in the stomach of the miniature pig.

### [Example 2]

A biofilm in a mouth (tooth surfaces) was cleaned off using the nozzle A in FIG. 1, the water jet spray nozzle 11 according to the embodiment of the present invention. For comparison with a typical water jet, a comparative experiment was conducted using the nozzle C in FIG. 2 (c) . In either of these cases, the nozzle was oriented so as to discharge a jet perpendicularly to tooth surfaces with a discharge pressure of 0.3 MPa, a standoff distance of 8 mm, an inside diameter of 0.5 mm at the small-diameter portion of the nozzle, and a discharge time of five minutes.

For clarification of a condition of the biofilm before cleaning, the stained biofilm was cleaned by the nozzle A and then was cleaned using the nozzle C in the air with a typical water jet. FIG. 8 shows a tooth surface condition at that time. FIG. 8(a) shows the results of a water jet discharge to the right incisor of an upper jaw through the nozzle A and a water jet discharge to the right incisor of a lower jaw through the nozzle C. FIG. 8(b) shows the results of a water jet discharge to the right incisor of the upper jaw through the nozzle A and a water jet discharge to the left incisor of the lower jaw through the nozzle C. FIG. 8(c) shows the results of a water jet discharge to the left incisor of the upper jaw through the nozzle A and a water jet discharge to the right incisor of the lower jaw through the nozzle C. FIGS. 8(a), 8(b), and 8(c) show different subjects.

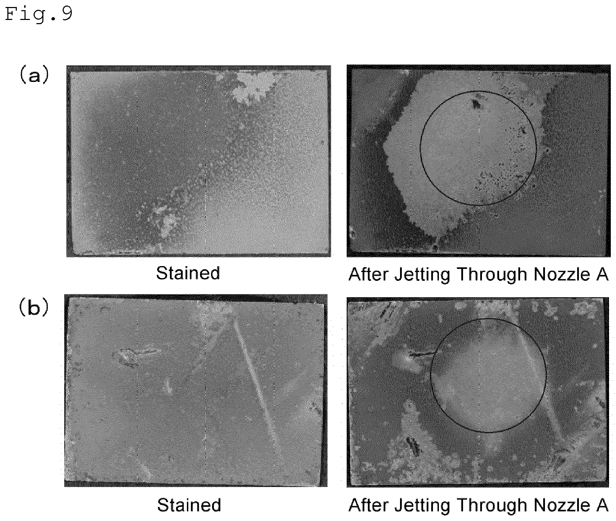

Moreover, a titanium test piece (5 mm × 7 mm) used for an implant was held in the mouth of a subject and then was removed to be subjected to a water jet for five minutes through the nozzle A (an inside diameter of 0.5 mm at the small-diameter portion) with a discharge pressure of 0.3 MPa and a standoff distance of 8 mm. For clarification of a condition of the biofilm, FIG. 9 shows a surface condition of the titanium test piece after the stained biofilm was cleaned by the nozzle A. FIGS. 9(a) and 9 (b) show different subjects.

As shown in FIGS. 8 and 9, it was confirmed that the water jet spray nozzle 11 according to the embodiment of the present invention can clean the biofilm in the mouth. Moreover, as shown in FIG. 8, it was confirmed that the water jet spray nozzle 11 according to the embodiment of the present invention has a more enhanced cleaning effect than a typical water jet.

### Reference Signs List

- 10: water jet spray device
- 11: water jet spray nozzle
- 11a: flow path
- 21: small-diameter portion
- 22: expanded portion
- 23: nozzle
- 12: supply unit

## Claims

1. A water jet spray nozzle(11) having a flow path(11a) including, from an upstream side to an end of the flow path(11a), a small-diameter portion(21) having a smaller inside diameter than the flow path upstream of the small-diameter portion(21), an expanded portion(22) having a larger inside diameter than the small-diameter portion(21), and a jet orifice(23) having a smaller inside diameter than the expanded portion(22) **characterized in that**,
the water jet spray nozzle(11) being configured such that water passing through the flow path(11a) with a predetermined pressure is retained in the expanded portion(22) so as to generate cavitation bubbles from the small-diameter portion(21) to the expanded portion(22); and
the inside diameter of the jet orifice(23) is 1 to 1.3 times larger than the inside diameter of the small-diameter portion(21); and
the inside diameter of the small-diameter portion(21) is 0.2 to 1.0 mm.

2. A water jet spray device(10) comprising:
the water jet spray nozzle(11) according to claim 1; and
a supply unit(12) that supplies water to the water jet spray nozzle(11) with the predetermined pressure.

3. The water jet spray device(10) according to claim 2, wherein the supply unit(12) is configured to supply water with a pressure of 0.2 to 0.6 MPa.

4. A gastrointestinal tract cleaning device, comprising:
the water jet spray nozzle(11) according to claim 1; and
a supply unit(12) that supplies water to the water jet spray nozzle(11) with a pressure of 0.2 to 0.6 MPa.

5. A gastric wall cleaning device, comprising:
the water jet spray nozzle(11) according to claim 1; and
a supply unit(12) that supplies water to the water jet spray nozzle(11) with a pressure of 0.2 to 0.6 MPa.

## Patentansprüche

1. Wasserstrahlsprühdüse (11) mit einem Strömungspfad (11a), der, von einer stromaufwärts gelegenen Seite bis zu einem Ende des Strömungspfads (11a) gesehen, einen Abschnitt mit einem kleinen Durchmesser (21) mit einem kleineren Innendurchmesser als der Strömungspfad stromaufwärts des Abschnitts mit einem kleinen Durchmesser (21), einen erweiterten Abschnitt (22) mit einem größeren Innendurchmesser als der Abschnitt mit dem kleinen Durchmesser (21) und eine Strahlöffnung (23) mit einem kleineren Innendurchmesser als der erweiterte Abschnitt (22) enthält, **dadurch gekennzeichnet, dass**
die Wasserstrahlsprühdüse (11) derart konfiguriert ist, dass Wasser, das mit einem vorbestimmten Druck durch den Strömungspfad (11a) fließt, in dem erweiterten Abschnitt (22) zurückgehalten wird, um Kavitationsblasen von dem Abschnitt mit dem kleinen Durchmesser (21) im erweiterten Abschnitt (22) zu erzeugen; und
dass der Innendurchmesser der Strahlöffnung (23) 1- bis 1,3-mal größer ist als der Innendurchmesser des Abschnitts mit dem kleinen Durchmesser (21); und
dass der Innendurchmesser des Abschnitts mit dem kleinen Durchmesser (21) 0,2 bis 1,0 mm beträgt.

2. Wasserstrahlsprühvorrichtung (10), umfassend:
die Wasserstrahlsprühdüse (11) nach Anspruch 1; und
eine Versorgungseinheit (12), die die Wasserstrahlsprühdüse (11) mit dem vorbestimmten Druck versorgt.

3. Wasserstrahlsprühvorrichtung (10) nach Anspruch 2, wobei die Versorgungseinheit (12) dazu konfiguriert ist, Wasser mit einem Druck von 0,2 bis 0,6 MPa bereitzustellen.

4. Reinigungsvorrichtung für den Magen-Darm-Trakt, umfassend:
die Wasserstrahlsprühdüse (11) nach Anspruch 1; und
eine Versorgungseinheit (12), die die Wasserstrahlsprühdüse (11) mit einem Druck von 0,2 bis 0,6 MPa versorgt.

5. Vorrichtung zur Reinigung der Magenwand, umfassend:
die Wasserstrahlsprühdüse (11) nach Anspruch 1; und
eine Versorgungseinheit (12), die die Wasserstrahlsprühdüse (11) mit einem Druck von 0,2 bis 0,6 MPa versorgt.

## Revendications

1. Buse de pulvérisation à jet d'eau (11) possédant une trajectoire d'écoulement (11a) comprenant, en allant d'un côté amont vers une extrémité de la trajectoire d'écoulement (11a), une portion de faible diamètre (21) possédant un plus petit diamètre intérieur que la trajectoire d'écoulement en amont de la portion de faible diamètre (21), une portion élargie (22) possédant un plus grand diamètre intérieur que la portion de faible diamètre (21), et un orifice de jet (23) possédant un plus petit diamètre intérieur que la portion élargie (22), **caractérisée en ce que** :
la buse de pulvérisation à jet d'eau (11) est configurée de manière à ce que l'eau qui passe dans la trajectoire d'écoulement (11a) à une pression prédéterminée soit retenue dans la portion élargie (22) de manière à produire des bulles de cavitation depuis la portion de faible diamètre (21) vers la portion élargie (22) ; et
le diamètre intérieur de l'orifice de jet (23) est 1 à 1,3 fois plus grand que le diamètre intérieur de la portion de faible diamètre (21) ; et
le diamètre intérieur de la portion de faible
diamètre (21) est de 0,2 à 1,0 mm.

2. Dispositif de pulvérisation à jet d'eau (10) comprenant :
la buse de pulvérisation à jet d'eau (11) selon la revendication 1 ; et
une unité d'alimentation (12) qui fournit à la buse de pulvérisation à jet d'eau (11) de l'eau à la pression prédéterminée.

3. Le dispositif de pulvérisation à jet d'eau (10) selon la revendication 2, dans lequel l'unité d'alimentation (12) est configurée pour fournir de l'eau à une pression de 0,2 à 0,6 MPa.

4. Dispositif de nettoyage du tractus gastrointestinal, comprenant :
la buse de pulvérisation à jet d'eau (11) selon la revendication 1 ; et
une unité d'alimentation (12) qui fournit à la buse de pulvérisation à jet d'eau (11) de l'eau à une pression de 0,2 à 0,6 MPa.

5. Dispositif de nettoyage de la paroi gastrique, comprenant :
la buse de pulvérisation à jet d'eau (11) selon la revendication 1 ; et
une unité d'alimentation (12) qui fournit à la buse de pulvérisation à jet d'eau (11) de l'eau à une pression de 0,2 à 0,6 MPa.
